# EUROPÄISCHE PATENTSCHRIFT

(11) **EP 0 871 437 B1**
(45) Veröffentlichungstag und Bekanntmachung des Hinweises auf die Patenterteilung: **17.05.2000**
(21) Anmeldenummer: 95942720.4
(22) Anmeldetag: 21.12.1995
(51) Int. Cl.: A61K 31/19, A61K 35/78

(54) **VERWENDUNG VON BOSWELLIASÄURE ZUR BEHANDLUNG VON HIRNTUMOREN**
USE OF BOSWELLIC ACID FOR TREATING BRAIN TUMOURS
UTILISATION D'ACIDE BOSWELLIQUE POUR LE TRAITEMENT DES TUMEURS DU CERVEAU

(30) Priorität: 21.12.1994 DE 4445728
(43) Veröffentlichungstag der Anmeldung: 21.10.1998
(73) Patentinhaber: Simmet, Thomas, 44801 Bochum (DE)
(72) Erfinder: SIMMET, Thomas, D-44801 Bochum (DE); AMMON, Hermann P. T., D-72072 Tübingen (DE)
(74) Vertreter: Kutzenberger, Helga, Dr.
(86) Internationale Anmeldenummer: EP9505073
(87) Internationale Veröffentlichungsnummer: WO9619212

(56) Entgegenhaltungen:
- WO-A-90/01937

## Beschreibung

Die Erfindung betrifft die Verwendung von reiner Boswelliasäure, eines physiologisch annehmbaren Salzes, eines Derivates, eines Salzes des Derivats oder einer Boswelliasäure enthaltenden pflanzlichen Zubereitung zur Herstellung eines Arzneimittels zur Behandlung von Hirntumoren.

Die Erfindung betrifft ebenfalls die Verwendung von reiner Boswelliasäure, eines physiologisch annehmbaren Salzes, eines Derivates, eines Salzes des Derivats oder einer Boswelliasäure enthaltenden pflanzlichen Zubereitung für die Behandlung von Hirntumoren.

Die bislang bekannten Möglichkeiten der therapeutischen Behandlung von Hirntumoren sind unbefriedigend:

Maligne Hirntumoren können bislang nur unzureichend behandelt werden. Die neurochirurgische Entfernung der Hirntumoren stellt einen schweren operativen Eingriff dar und führt, je nach Art, Größe und Lage der Hirntumoren, oftmals zu keiner vollständigen Entfernung der malignen Tumore. Aus diesen Gründen beträgt die mittlere Überlebenszeit der Patienten mit malignen Hirntumoren auch nach Kombinationsbehandlung mit Operation und Strahlentherapie nur circa 9 Monate. Eine zusätzliche Chemotherapie mit bislang verfügbaren Zytostatika verlängert die Überlebensdauer lediglich um circa 10% (Lesser, G.J., Grossman S., The chemotherapy of high-grade astrocytomas, Seminars in Oncology, 1994, 21:220-235).

Zur symptomatischen Behandlung werden Glucocorticosteroide eingesetzt, die jedoch die peritumoralen Hirnödeme nicht effektiv vermindern können, so daß ihre Anwendung nicht zum gewünschten Erfolg führt.

Der Erfindung liegt daher die Aufgabe zugrunde, die Verwendung von Präparaten zur Verfügung zu stellen, die zur Behandlung von Hirntumoren, zur Hemmung des peritumoralen Hirnödems sowie des Tumorzellwachstums und zum Abtöten der Tumorzellen dienen. Insbesondere soll ein Präparat bereitgestellt werden, das eine wesentlich wirksamere Behandlung von Hirntumoren ermöglicht, ohne dabei die Nebenwirkungen und die geringe Effizienz aufzuweisen, die mit der Verwendung bislang zur Tumortherapie eingesetzter Zytostatika einhergehen. Das erfindungsgemäß zur Verfügung gestellte Arzneimittel bzw. Präparat soll eine geringe Toxizität besitzen und dementsprechend von den Patienten gut toleriert werden. Die Bereitstellung eines solchen Arzneimittels ist Gegenstand zahlreicher Untersuchungen (De Vita, D., Helman, S., Rosenberg, S.A. (Hrsg.), Cancer - Principles and Practice of Oncology, 4. Auflage, 1993, J.B. Lippincott Company, Philadelphia; Lesser, G.J., Grossman S., The chemotherapy of high-grade astrocytomas, Seminars in Oncology, 1994, 21:220-235), die das dringende Bedürfnis nach der Schaffung eines solchen Arzneimittels dokumentieren.

Überraschenderweise wurde jetzt gefunden, daß Boswelliasäure, ein physiologisch annehmbares Salz, ein Derivat, ein Salz des Derivats oder eine Boswelliasäure enthaltende pflanzliche Zubereitung für die Behandlung von Hirntumoren wirksam ist.

In der ayurvedischen Medizin Indiens werden Arzneimittel, die Präparationen aus der Pflanze Boswellia serrata enthalten, zur Behandlung von Entzündungen, aber auch Rheumatismus verwendet. Hinweise, diese Arzneimittel zur Behandlung von Hirntumoren einzusetzen, finden sich jedoch in der Literatur nicht. Aufgrund der Wirksamkeit von Präparationen aus der Pflanze Boswellia serrata zur Behandlung von entzündlichen Krankheiten wurde dieses Harz bereits auf seine Inhaltsstoffe hin untersucht. So berichten Pardhy & Bhattacharyya (Ind. J. Chem., 16B:176-178, 1978), daß Boswellia serrata im wesentlichen die folgenden Inhaltsstoffe enthält:
β-Boswelliasäure, Acetyl-β-boswelliasäure, Acetyl-11-keto-β-boswelliasäure, 11-Keto-β-boswelliasäure.

Bislang sind jedoch noch keine Untersuchungen über die Wirksamkeit dieser Verbindungen zur Behandlung von Hirntumoren bekannt.

Die Strukturformeln von Boswelliasäure und einigen ihrer Derivate werden im folgenden aufgeführt: 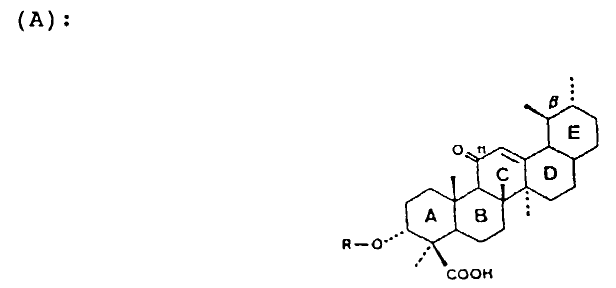
- R = H :: 11-Keto-β-boswelliasäure
- R = Acetyl :: Acetyl-11-keto-β-boswelliasäure
- R = Formyl :: Formyl-11-keto-β-boswelliasäure 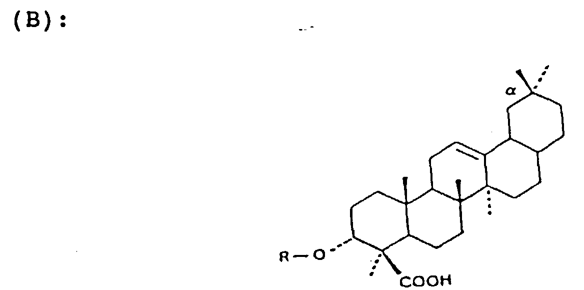
- R = H :: α-Boswelliasäure
- R = Acetyl :: Acetyl-α-boswelliasäure
- R = Formyl :: Formyl-α-boswelliasäure

Als Boswelliasäure wird vorzugsweise β-Boswelliasäure verwendet, die nach Literaturangaben aus Boswellia serrata oder anderen bekannten Boswelliasäure enthaltenden Pflanzen isoliert wird. Die β-Boswelliasäure kann geringe Mengen an α-oder γ-Boswelliasäure enthalten. Als physiologisch annehmbare Salze der Boswelliasäure können die Natrium-, Kalium-, Ammonium-, Calciumsalze verwendet werden. Als Derivate der Boswelliasäure können niedere Alkylester, die durch Veresterung der Carboxylgruppe mit einem C₁-C₆-Alkohol erhalten werden, vorzugsweise der Methylester, oder Ester, die durch Veresterung der Hydroxylgruppe mit einer physiologisch verträglichen Carbonsäure erhalten werden, verwendet werden. Bevorzugte Derivate sind β-Boswelliasäureacetat, β-Boswelliasäureformiat, β-Boswelliasäuremethylester, Acetyl-β-boswelliasäure, Acetyl-11-keto-β-boswelliasäure und 11-Keto-β-boswelliasäure.

Erfindungsgemäß ist es weiterhin möglich, eine Boswelliasäure enthaltende pflanzliche Zubereitung zu verwenden. Erfindungsgemäß werden Präparate, die aus dem Harz von Boswellia-Arten (Olibanum, Weihrauch) gewonnen werden, verwendet.

Pflanzen, die Boswelliasäure (syn.: Boswellinsäure) enthalten, sind:

Boswellia (serrata, papyrifera, frereana, carteri, thurifera, glabra, bhaw-dajiana, oblongata, socotrana und andere Vertreter dieser Familie).

Ein ethanolischer Extrakt aus dem Harz der Boswellia serrata, der die genannten Boswelliasäuren enthält, erweist sich als besonders wirksam: Die Anwendung dieses Präparats - im weiteren als Phytopharmakon H 15 bezeichnet (hergestellt und vertrieben von der Firma Ayurmedica, Pöcking) - ermöglicht innerhalb einer siebentägigen Behandlungsdauer eine Senkung des peritumoralen Hirnödems um 22 bis 48 %. Eine histopathologische Untersuchung des Tumorgewebes der behandelten Patienten erweist sich als weitestgehend nekrotisch, was auβerordentlich ungewöhnlich ist. Die Lebensfähigkeit der explantierten Zellen liegt außerordentlich niedrig und beträgt in Zellkultur etwa 3,5 bis 4,5 %. Normalerweise beträgt die Lebensfähigkeit solcher Zellen etwa 80 %. Im Gegensatz zu den Zellen unbehandelter Patienten zeigen die Zellen des behandelten Tumorgewebes über zwei Wochen keinerlei Proliferationstendenz.

Diese Untersuchungen zeigen, daß H 15 das peritumorale Hirnödem sowie das Tumorzellwachstum hemmt und zum Absterben der Tumorzellen führt.

Erfindungsgemäß ist es weiterhin möglich, daß die Verwendung zusammen mit anderen chemisch reinen Arzneistoffen und/oder anderen pflanzlichen Arzneimitteln erfolgt. Beispiele für solche andere chemisch reine Arzneistoffe sind:

Zytostatika, z.B. Nimustin, Carmustin, Lomustin, Methyllomustin, Semustin, Methotrexat, Teniposid, Dacarbazin, Procarbazin, Temozolamid, Topotecan, Paclitaxel, Streptozocin, Cisplatin, 5-Fluorouracil; Glucocorticosteroide, z.B. Dexamethason, Prednisolon, Methylprednisolon, Prednison, Hydrocortison, Cloprednol, Betamethason.

Ein Beispiel für solche pflanzliche Arzneimittel ist Vincristin.

Erfindungsgemäß wird die Boswelliasäure je nach Bedarf verabreicht. Da sie wenig toxisch ist, ist die Dosierung nicht kritisch und kann in Abhängigkeit von der Schwere der Krankheit, dem Gewicht des zu behandelnden Patienten und der Dauer der Behandlung vom Arzt leicht variiert werden.

Einheitsdosen können beispielsweise ein- bis viermal täglich verabreicht werden. Die exakte Dosis hängt vom Verabreichungsweg und dem zu behandelnden Zustand ab. Naturgemäß kann es erforderlich sein, Routinevariationen der Dosis, je nach dem Alter und dem Gewicht des Patienten sowie der Schwere des zu behandelnden Krankheitszustandes, vorzunehmen.

Die erfindungsgemäß verwendeten Zubereitungen können in an sich bekannter Weise unter Verwendung eines oder mehrerer pharmazeutisch annehmbarer Träger oder Verdünnungsmittel formuliert werden. Die Zubereitungen können für die orale, parenterale, rektale, intrakranielle oder intrathekale Verabreichung formuliert werden. Zubereitungen der Verbindungen für die orale Verabreichung sind bevorzugt.

Die pharmazeutischen Zubereitungen für die orale Verabreichung können in Form von beispielsweise Tabletten oder Kapseln, die nach an sich bekannten Verfahren mit pharmazeutisch annehmbaren Verdünnungsmitteln, wie Bindemitteln (zum Beispiel vorgelatinierter Maisstärke, Polyvinylpyrrolidon oder Hydroxypropylmethylcellulose), Füllstoffen (zum Beispiel Lactose, Saccharose, Mannit, Maisstärke, mikrokristalline Cellulose oder Calciumhydrogenphosphat); Schmiermitteln (zum Beispiel Stearinsäure, Polyethylenglykol, Magnesiumstearat, Talk oder Siliciumdioxid); Desintegrationsmitteln (zum Beispiel Kartoffelstärke, Natriumstärkeglykolat oder Natriumcarboxymethylcellulose); oder Benetzungsmitteln (zum Beispiel Natriumlaurylsulfat), hergestellt werden, vorliegen. Die Tabletten können nach an sich bekannten Verfahren überzogen werden. Flüssige Zubereitungen für die orale Verabreichung können in Form von beispielsweise wäßrigen oder öligen Lösungen, Sirupen, Elixieren, Emulsionen oder Suspensionen vorliegen, oder sie können als Trockenprodukt für die Konstitution mit Wasser oder einem anderen geeigneten Träger vor der Verwendung vorliegen. Solche flüssigen Zubereitungen können nach an sich bekannten Verfahren mit pharmazeutisch annehmbaren Zusatzstoffen hergestellt werden, wie Suspensionsmitteln (zum Beispiel Sorbitsirup, Cellulosederivaten, Glucose/Zucker-Sirup, Gelatine, Aluminiumstearatgel oder hydrierten genießbaren Fetten); Emulgiermitteln (zum Beispiel Lecithin, Gummi arabicum oder Sorbitan-monooleat); nichtwäßrigen Trägern (zum Beispiel Mandelöl, öligen Estern, Ethylalkohol oder fraktionierten Pflanzenölen); und Konservierungsmitteln (zum Beispiel Methyl- oder Propyl-p-hydroxybenzoaten oder Sorbinsäure). Die flüssigen Zubereitungen können auch an sich bekannte Puffer, Geschmacks- bzw. Aromamittel, Farbstoffe und Süßstoffe, je nach Bedarf, enthalten.

Für die parenterale Verabreichung können die Verbindungen für Injektionen, bevorzugt intravenöse, intraarterielle, intramuskuläre, intrakranielle, intrathekale oder subkutane Injektionen, formuliert werden. Zubereitungen für die Injektion können in Eindosenform, zum Beispiel in Ampullen, oder in Mehrfachdosis-Behältern mit einem zugegebenen Konservierungsstoff vorliegen. Die Zubereitungen können in Form von Suspensionen, Lösungen oder Emulsionen in öligen oder wäßrigen Trägern vorliegen und Zubereitungshilfsmittel enthalten, wie Suspendier-, Stabilisier- und/oder Dispersionsmittel, und/oder Mittel zur Einstellung der Tonizität der Lösung enthalten. Alternativ kann der aktive Bestandteil in Pulverform für die Konstitution mit einem geeigneten Träger, zum Beispiel sterilem pyrogenfreien Wasser, vor der Verwendung vorliegen.

Die Verbindungen können ebenfalls als rektale Zubereitungen, wie Suppositorien, formuliert werden, zum Beispiel solche, die an sich bekannte Suppositorien-Grundstoffe, wie Kakaobutter oder andere Glyceride, enthalten.

Die folgenden Beispiele erläutern die erfindungsgemäße Verwendung.

### Beispiel 1

| Tabletten für die orale Verabreichung | |
|---|---|
| A. Direkte Kompression | |
| (1) | |
| Wirkstoff: Boswelliasäure | 15 - 30 mg/Tablette |
| (bzw. pulverisierte Droge | 0,5 - 1,0 g/Tablette) |
| | |
| Magnesiumstearat BP | 0,65 mg/Tablette |
| | |
| wasserfreie Lactose | 80 mg/Tablette |

Der Wirkstoff wird mit der wasserfreien Lactose und dem Magnesiumstearat vermischt, und das Gemisch wird gesiebt. Das entstehende Gemisch wird zu Tabletten unter Verwendung einer Tablettiermaschine verpreßt.

| (2) | |
|---|---|
| Wirkstoff: Boswelliasäure | 15 - 30 mg/Tablette |
| (bzw. pulverisierte Droge | 0,5 - 1,0 g/Tablette) |
| | |
| Magnesiumstearat BP | 0,7 mg/Tablette |
| | |
| mikrokristalline Cellulose NF | 100 mg/Tablette |

Der Wirkstoff wird gesiebt und mit der mikrokristallinen Cellulose und dem Magnesiumstearat vermischt. Das entstehende Gemisch wird unter Verwendung einer Tablettiermaschine zu Tabletten verpreßt.

| B. Nasse Granulierung | |
|---|---|
| Wirkstoff: Boswelliasäure | 15 - 30 mg/Tablette |
| (bzw. pulverisierte Droge | 0,5 - 1,0 g/Tablette) |
| | |
| Lactose BP | 150,0 mg/Tablette |
| | |
| Stärke BP | 30,0 mg/Tablette |
| | |
| vorgelatinierte Maisstärke BP | 15,0 mg/Tablette |
| | |
| Magnesiumstearat BP | 1,5 mg/Tablette |

Der Wirkstoff wird durch ein geeignetes Sieb gesiebt und mit der Lactose, der Stärke und der vorgelatinierten Maisstärke vermischt. Geeignete Volumina an gereinigtem Wasser werden zugegeben, und das Pulver wird granuliert. Nach dem Trocknen wird das Granulat gesiebt und mit dem Magnesiumstearat vermischt. Das Granulat wird dann unter Verwendung von Lochstanzen mit geeignetem Durchmesser zu Tabletten verpreßt. Tabletten anderer Zusammensetzung können hergestellt werden, indem man das Verhältnis von Wirkstoff zu Lactose oder das Kompressionsgewicht ändert und entsprechende Lochstanzen verwendet.

### Beispiel 2

| Kapseln | |
|---|---|
| Wirkstoff: Boswelliasäure | 15 - 30 mg/Kapsel |
| (bzw. granulierte Droge | 0,5 - 1,0 g/Kapsel |
| | |
| freifließende Stärke | 150,00 mg/Kapsel |
| | |
| Magnesiumstearat BP | 1,00 mg/Kapsel |

Der Wirkstoff wird gesiebt und mit den anderen Bestandteilen vermischt. Das Gemisch wird unter Verwendung einer geeigneten Vorrichtung in Hartgelatinekapseln Nr. 2 gefüllt. Andere Kapseln können hergestellt werden, indem man das Füllgewicht ändert und erforderlichenfalls die Kapselgröße entsprechend ändert.

### Beispiel 3

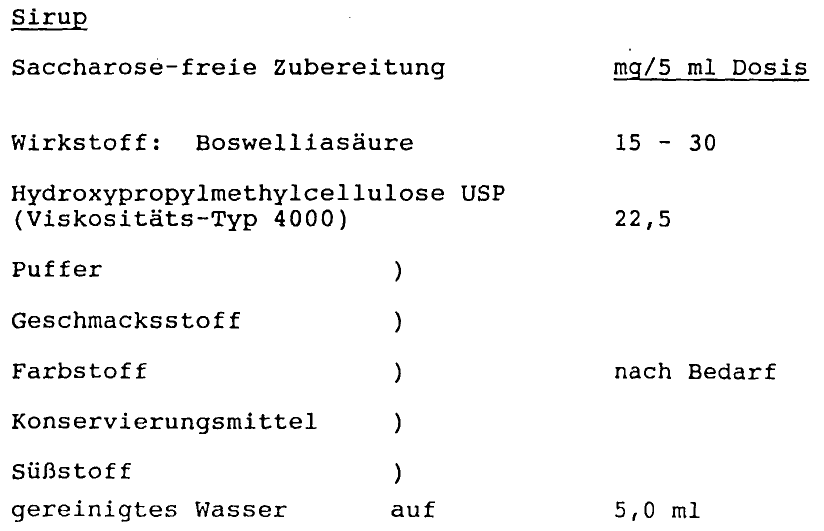

Die Hydroxypropylmethylcellulose wird in heißem Wasser dispergiert, abgekühlt und dann mit einer wäßrigen Suspension vermischt, die den Wirkstoff und die anderen Komponenten der Zubereitung enthält. Die entstehende Lösung wird auf ihr Volumen eingestellt und vermischt.

### Beispiel 4

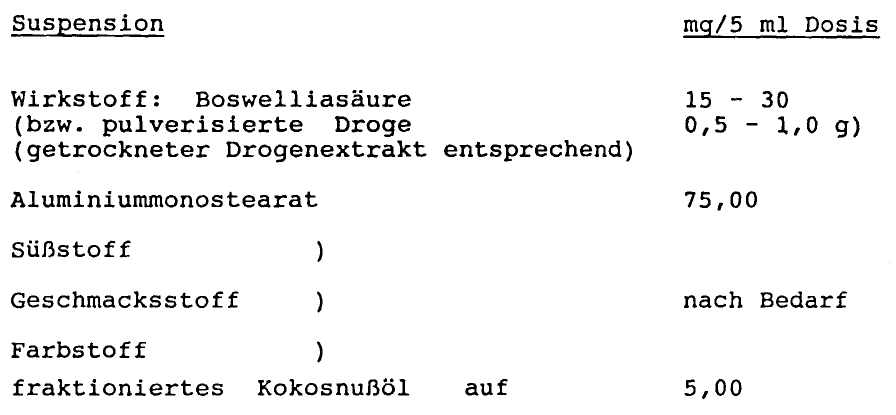

Das Aluminiummonostearat wird in etwa 90% des fraktionierten Kokosnußöls dispergiert. Die resultierende Suspension wird unter Rühren auf 115°C erhitzt und dann abgekühlt. Die Süß-, Geschmacks- und Farbstoffe werden zugesetzt, und der Wirkstoff wird dispergiert. Die Suspension wird mit dem restlichen fraktionierten Kokosnußöl auf das Volumen eingestellt und vermischt.

### Beispiel 5

| Sublinguale Tablette | |
|---|---|
| Wirkstoff: Boswelliasäure | 15 - 30 mg/Tablette |
| (bzw. Drogenextrakt | 0,5 - 1,0 g/Tablette) |
| | |
| verpreßbarer Zucker NF | 50,5 mg/Tablette |
| | |
| Magnesiumstearat BP | 0,5 mg/Tablette |

Der Wirkstoff wird durch ein geeignetes Sieb gesiebt, mit den anderen Bestandteilen vermischt und unter Verwendung ge eigneter Lochstanzen verpreßt. Tabletten anderer Stärke kön nen hergestellt werden, indem man das Verhältnis von Wirkstoff zu Träger oder das Kompressionsgewicht ändert.

### Beispiel 6

| Suppositorien für die rektale Verabreichung | |
|---|---|
| Wirkstoff: Boswelliasäure | 15 - 30 mg |
| Witepsol H15⁺ auf | 1,0 g |
| ⁺ geeignete Qualität von Adeps solidus Ph.Eur. | |

Eine Suspension des Wirkstoffs in geschmolzenem Witepsol wird hergestellt und unter Verwendung einer geeigneten Vorrichtung in 1-g-Suppositorienformen eingefüllt.

### Beispiel 7

| Injektion für intravenöse Verabreichung | |
|---|---|
| Wirkstoff: Boswelliasäure | 15 - 30 mg/ml |
| | |
| Natriumchlorid-intravenöse Infusion, BP, 0,9% Gew./Vol. auf | 1 ml |
| Ansatzgröße 2500 ml | |

Der Wirkstoff wird in einem Teil der Natriumchlorid-intravenösen Infusion gelöst, die Lösung mit der Natriumchloridintravenösen Infusion auf das Volumen eingestellt und die Lösung gründlich vermischt. Die Lösung wird in klare, Typ 1, 10-ml-Glasampullen eingefüllt und unter Stickstoff im Kopfraum durch Abschmelzen des Glases abgesiegelt. Die Ampullen werden durch Erhitzen im Autoklaven bei 120°C für nicht kürzer als 20 Minuten sterilisiert.

## Patentansprüche

1. Verwendung von reiner Boswelliasäure, eines physiologisch annehmbaren Salzes, eines Derivates, eines Salzes des Derivats oder einer Boswelliasäure enthaltenden pflanzlichen Zubereitung zur Herstellung eines Arzneimittels zur Behandlung von Hirntumoren und zur Hemmung peritumoraler Hirnödeme

2. Verwendung nach Anspruch 1, dadurch **gekennzeichnet**, daß ein Arzneimittel zur Hemmung des Tumorzellwachstums und zum Abtöten der Tumorzellen hergestellt wird.

3. Verwendung nach den Ansprüchen 1 oder 2, dadurch **gekennzeichnet**, daß man als die Boswelliasäure enthaltende pflanzliche Zubereitung einen Weihrauchextrakt einsetzt.

4. Verwendung nach einem der Ansprüche 1 bis 3, dadurch **gekennzeichnet**, daß die Verwendung zur Herstellung eines Arzneimittels für die orale, bukkale, rektale, intramuskuläre, subkutane, intraartikuläre, intravenöse, intrakranielle oder intrathekale Verabreichung erfolgt.

5. Verwendung nach einem der Ansprüche 1 bis 4, dadurch **gekennzeichnet**, daß die Verwendung zur Herstellung eines Arzneimittels in Form von Tabletten, Dragees, Kapseln, Lösungen, polymergebundenen Präparationen oder Suppositorien erfolgt.

6. Verwendung nach einem der Ansprüche 1 bis 5, dadurch **gekennzeichnet**, daß die Verwendung zusammen mit anderen chemisch reinen Arzneistoffen und/oder pflanzlichen Arzneimitteln erfolgt.

7. Verwendung nach Anspruch 6, dadurch **gekennzeichnet**, daß die Verwendung zusammen mit Zytostatika und/oder Glucocorticosteroiden erfolgt.

8. Verwendung von reiner Boswelliasäure, eines physiologisch annehmbaren Salzes, eines Derivates, eines Salzes des Derivats oder einer Boswelliasäure enthaltenden pflanzlichen Zubereitung für die Behandlung von Hirntumoren und zur Hemmung peritumoraler Hirnödeme.

9. Verwendung nach Anspruch 8, dadurch **gekennzeichnet**, daß die Verwendung zur Hemmung des Tumorzellwachstums und zum Abtöten von Tumorzellen erfolgt.

10. Verwendung nach einem der Ansprüche 8 oder 9, dadurch **gekennzeichnet**, daß man als die Boswelliasäure enthaltende pflanzliche Zubereitung einen Weihrauchextrakt einsetzt.

11. Verwendung nach einem der Ansprüche 7 bis 10, dadurch **gekennzeichnet**, daß die Verwendung durch orale, bukkale, rektale, intramuskuläre, subkutane, intraartikuläre, intravenöse, intrakranielle oder intrathekale Verabreichung erfolgt.

12. Verwendung nach einem der Ansprüche 7 bis 11, dadurch **gekennzeichnet**, daß die Verwendung in Form von Tabletten, Dragees, Kapseln, Lösungen, polymergebundenen Präparationen oder Suppositorien erfolgt.

## Claims

1. The use of pure boswellic acid, a physiologically acceptable salt, a derivative, a salt of said derivative or a vegetable preparation containing boswellic acid for the preparation of a pharmaceutical composition for the treatment of brain tumours and for the inhibition of peritumoural brain oedemas.

2. The use according to claim 1,
**characterized** in that
a pharmaceutical composition is prepared for inhibiting the growth of tumour cells as well as for destroying the tumour cells.

3. The use according to claims 1 or 2,
**characterized** in that
an incense extract is used as the vegetable preparation containing boswellic acid.

4. The use according to any of claims 1 to 3,
**characterized** in that
said use is for preparing a pharmaceutical composition for oral, buccal, rectal, intramuscular, subcutaneous, intraarticular, intravenous, intracranial, or intrathecal administration.

5. The use according to any of claims 1 to 4,
**characterized** in that
said use is for preparing a pharmaceutical composition in the form of tablets, dragees, capsules, solutions, polymer-bound preparations, or suppositories.

6. The use according to any of claims 1 to 5,
**characterized** in that
said use is together with other chemically pure drugs and/or vegetable drugs.

7. The use according to claim 6,
**characterized** in that
said use is together with cytostatics and/or glucocorticosteroids.

8. The use of pure boswellic acid, a physiologically acceptable salt, a derivative, a salt of said derivative or a vegetable preparation containing boswellic acid for the treatment of brain tumours and for the inhibition ofperitumoural brain oedemas.

9. The use according to claim 8,
**characterized** in that
said use is for inhibiting the growth of tumour cells and for destroying the tumour cells.

10. The use according to any of claims 8 or 9,
**characterized** in that
an incense extract is used as the vegetable preparation containing boswellic acid.

11. The use according to any of claims 7 to 10,
**characterized** in that
said use is by oral, buccal, rectal, intramuscular, subcutaneous, intraarticular, intravenous, intracranial, or intrathecal administration.

12. The use according to any of claims 7 to 11,
**characterized** in that
said use is in the form of tablets, dragees, capsules, solutions, polymer-bound preparations or suppositories.

## Revendications

1. Utilisation d'acide boswellique pur d'un sel physiologiquement acceptable, d'un dérivé, d'un sel du dérivé ou d'une préparation végétale contenant de l'acide boswellique pour préparer un médicament pour le traitement des tumeurs de l'encéphale et pour inhiber les oedèmes péritumoraux de l'encéphale.

2. Utilisation selon la revendication 1, caractérisée en ce qu'on prépare un médicament pour inhiber la croissance des cellules tumorales et pour tuer les cellules tumorales.

3. Utilisation selon les revendications 1 ou 2, caractérisée en ce que l'on utilise comme préparation végétale contenant de l'acide boswellique, un extrait d'encens.

4. Utilisation selon l'une des revendications 1 à 3, caractérisée en ce que l'utilisation s'effectue pour la préparation d'un médicament administré par voie orale, buccale, rectale, intramusculaire, sous-cutanée, intraarticulaire, intraveineuse, intracranienne ou intrathécale.

5. Utilisation selon l'une des revendications 1 à 4, caractérisée en ce que l'utilisation s'effectue pour la préparation d'un médicament sous la forme de comprimés, de dragées, de capsules, de solutions, de préparations liées par un polymère ou de suppositoires.

6. Utilisation selon l'une des revendications 1 à 5, caractérisée en ce que l'utilisation s'effectue avec d'autres médicaments purs chimiques et/ou des préparations médicamenteuses végétales.

7. Utilisation selon la revendication 6, caractérisée en ce que l'utilisation s'effectue avec des cytostatiques et/ou des glucocorticostéroïdes.

8. Utilisation d'acide boswellique pur, d'un sel physiologiquement acceptable, d'un dérivé, d'un sel des dérivés ou d'une préparation végétale contenant de l'acide boswellique pour le traitement des tumeurs de l'encéphale et pour inhiber les oedèmes péritumoraux de l'encéphale.

9. Utilisation selon la revendication 8, caractérisée en ce que l'utilisation s'effectue pour inhiber la croissance des cellules tumorales et pour tuer les cellules tumorales.

10. Utilisation selon l'une des revendications 8 ou 9, caractérisée en ce que l'on utilise comme préparation végétale contenant de l'acide boswellique, un extrait d'encens.

11. Utilisation selon l'une des revendications 7 à 10, caractérisée en ce que l'utilisation s'effectue par administration orale, buccale, rectale, intramusculaire, sous-cutanée, intra-articulaire, intraveineuse, intracranienne ou intrathécale.

12. Utilisation selon l'une des revendications 7 à 11, caractérisée en ce que l'utilisation s'effectue sous forme de comprimés, de dragées, de capsules, de solutions, de préparations liées par un polymère ou de suppositoires.
